# EUROPEAN PATENT SPECIFICATION

(11) **EP 2 878 336 B1**
(45) Date of publication and mention of the grant of the patent: **30.10.2019**
(21) Application number: 14192455.5
(22) Date of filing: 10.11.2014
(51) Int. Cl.: A61B 90/50, A61N 2/02, A61N 2/00

(54) **Device support apparatus**
Vorrichtungsträgervorrichtung
Appareil de support de dispositif

(30) Priority: 29.11.2013 FI 20136201; 29.11.2013 US 201314093073; 23.12.2013 GB 201322845
(43) Date of publication of application: 03.06.2015
(73) Proprietor: Nexstim Oy, 00510 Helsinki (FI)
(72) Inventor: Hynninen, Pentti, 00510 Helsinki (FI); Järnefelt, Gustaf, 00510 Helsinki (FI)
(74) Representative: Seppo Laine Oy

(56) References cited:
- US-A1- 2006 161 039
- US-A1- 2008 262 287
- US-A1- 2009 142 724
- US-A1- 2009 227 830

## Description

### FIELD OF INVENTION

The present invention relates to positioning, fixing and/or moving devices with respect to persons seated in a chair.

### BACKGROUND OF INVENTION

Performing precision operations with instruments requires that the instruments are accurately positioned with respect to whatever the operations are being performed on. Transcranial magnetic stimulation, TMS, for example, is used to stimulate a small area inside a person's brain which requires that the magnetic coil used be positioned accurately, for shorter or longer periods of time, along the outside of the person's head. Similarly, for example, radiation therapy greatly benefits from aiming radiation patterns accurately at malignant cells to avoid damaging healthy tissue.

Using TMS as an example, a patient may be instructed to maintain his head immobile while a stimulating coil is moved along the outer surface of his head. If the coil is held accurately and reliably at the correct location, the stimulating effect can be aimed at a desired location inside the brain. On the other hand if the coil is inaccurately placed or accidentally moves, results of TMS are expected to be adversely affected.

TMS may be used, for example, to locate a person's motor threshold position, to facilitate rehabilitation following a stroke or to treat depression. Radiation therapy may be used to kill malignant cells. Using a manual placement technique, a treatment position on the patient's head or a position used to find a treatment position, such as the patient's motor threshold position, MTP, may be determined by moving a TMS coil near a predicted area determined by patient anatomical landmarks until the desired motor response is achieved.

Document US2009227830 describes an apparatus for positioning a medical instrument, such as a TMS coil, with respect to a patient. Document US2008/262287 discloses a system for magnetic stimulation, wherein a lockable articulated arm is mounted on a straight rail.

### SUMMARY OF THE INVENTION

The invention is defined by the appended independent claim. Preferred embodiments of the invention are illustrated in the dependent claims. According to a first aspect of the present invention, there is provided an apparatus, comprising a first arm configured to accept a transcranial magnetic stimulation device, the first arm comprising a first counterweight arrangement, a second arm supporting the first arm by a coupling, and a base defining and being formed in a shape of a non-straight curve and having the second arm mounted thereon at an attachment point, wherein the attachment point is movable along the non-straight curve to enable positioning the transcranial magnetic stimulation device to different sides of a head, the base configured to be mounted on a chair.

In some embodiments, the first counterweight arrangement comprises that cabling of the device is attached to the first arm.

In some embodiments, the first counterweight arrangement comprises a display screen attached to the first arm.

According to a second aspect of the present invention, there is provided a system comprising an apparatus according to the first aspect attached to a chair, wherein the system is a transcranial magnetic stimulation system.

### BRIEF DESCRIPTION OF THE DRAWINGS

FIGURE 1 illustrates an example apparatus in accordance with at least some embodiments of the invention.
FIGURE 2 illustrates the example apparatus of FIGURE 1 from a different perspective, and
FIGURE 3 illustrates another example apparatus in accordance with at least some embodiments of the invention.

### DETAILED DESCRIPTION OF EXEMPLARY EMBODIMENTS

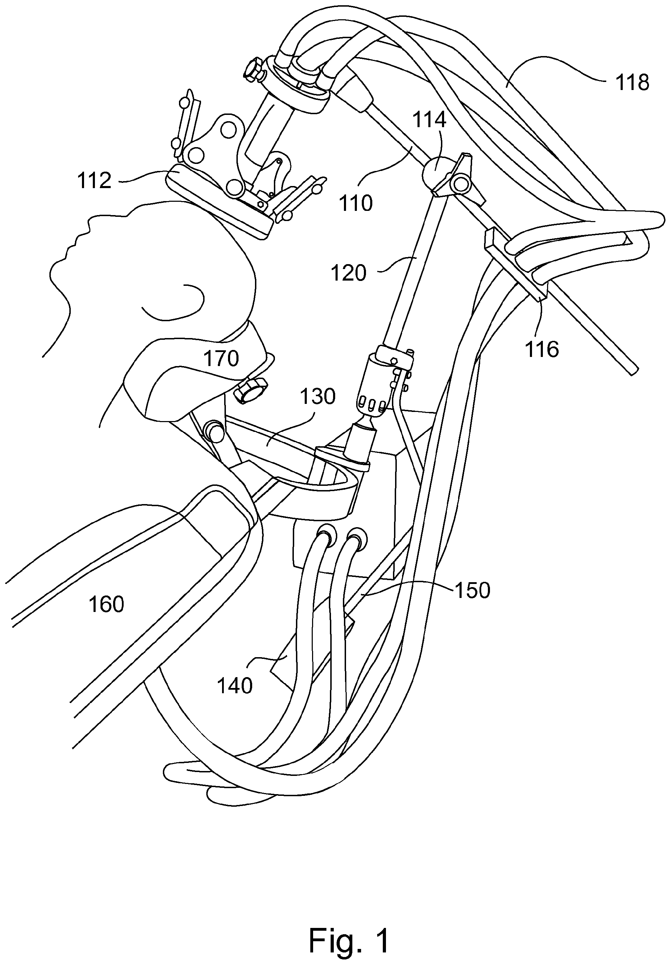

FIGURE 1 illustrates an example apparatus in accordance with at least some embodiments of the invention. The example of FIGURE 1 may relate to a TMS system suitable for investigating anatomical features of a person's brain using electromagnetic fields, or treating the brain, for example. Illustrated is chair 160, which may comprise a reclining treatment chair suitable for TMS or radiation therapy, for example. On chair 160 is disposed headrest 170, which may be contoured to assist a person sitting in chair 160 to maintain his head immobile. To such effect, headrest 170 may be at least in part comprised of a material that accepts and retains a shape the back of a person's head, so the head can more easily be maintained stationary with respect to the chair.

An operator may place a TMS coil, for example, next to a person's head by hand and search for specific areas of the brain by monitoring for reactions responsive to magnetic stimuli. Holding a coil by hand for any period of time will tire the operator's hand even in case the coil is lightweight. When tired the operator becomes less capable of precisely holding the coil in place and the results of TMS are affected. Also in case a break is needed during a TMS session, continuing may be difficult in case the exact place where the session was interrupted is not immediately findable.

Accuracy requirements in TMS or radiation therapy may vary depending on the application. TMS may require an accuracy of at least 2 millimeters or 2 degrees, for example. An accuracy requirement of at least 2 millimeters may mean that a measurement needs to be capable of determining a position of device 112 with a measurement error of at most 2 millimeters. Alternatively, an accuracy requirement of 2 millimeters may mean that a location in a brain needs to be determined such that an error in the location in the brain is at most 2 millimeters. Accuracy requirements of radiation therapy may be substantially similar to that of TMS, as even larger tumours have edges that may be in direct contact with healthy tissue.

Device 112 may be operable to emit particulate or electromagnetic beams to the head of a person sitting in chair 160. Device 112 may comprise a TMS coil, wherein the coil may be enabled to generate an electromagnetic field at a distance from the coil inside the head. A TMS coil may be a coil winding device including a casing and contained in the casing coil windings of an electrically conductive material. A TMS system may comprise a computerized electromechanical instrument that produces and delivers brief duration rapidly alternating, or pulsed, magnetic fields to induce electrical currents directed at localized regions of the cortex. Device 112 may be powered by electrical cabling 118, which may be connected to electrical power and control devices. A TMS coil may be constructed to generate a magnetic field of known shape.

Cabling 118 may comprise one or more leads connecting device 112 to one or more units. Cabling 118 may provide to device 112 electrical energy and, optionally, control signals, for example signals to begin emitting electromagnetic radiation, or to cease emitting electromagnetic radiation. Cabling 118 may provide information of the functioning of device 112, for example cabling 118 may provide information on operational parameters of device 112. Cabling 118 may provide information on error conditions device 112 may find itself in.

Device 112 is in the illustrated example mounted at a first end of first arm 110 on a device attachment portion, for example. An operator of device 112 may move device 112 along the surface of the person's head. First arm 110 may be supported on second arm 120 by coupling 114. Coupling 114 may be configured to allow first arm 110 to move in a sliding manner through coupling 114, and/or coupling 114 may be configured to allow first arm 110 to rotate about a section of first arm 110 attached to coupling 114. In general the coupling may be arranged to provide for one or more than one degree of freedom of movement for the first arm with respect to the second arm. Device 112 may be attached to the first end of first arm 110 and/or the device attachment portion via a ball joint, for example, to provide for degrees of freedom for maneuvering device 112 along the surface of the head. The ball joint may be lockable individually. The ball joint may be electrically lockable by a mechanism wherein a single actuator is configured to lock all joints of the apparatus. In some embodiments, coupling 114 comprises a ball joint, hinge or bearing.

Attached to first arm 110 may be a connecting unit 116. Connecting unit 116 may be arranged to provide for a connection of cabling 118, so that the weight of cabling 118 forms a first counterweight, to the weight of device 112 with respect to coupling 114. Connecting unit 116 may be referred to as a cable attachment portion. This makes moving device 112 easier and lighter, which provides for improved accuracy in radiation treatment or TMS. An operator's hand becomes less tired when holding and moving device 112 due to the counterweight. Additionally, should the operator let go of device 112, counterweighting may keep device 112 stationary. In other words, device 112 may be left in its position simply by letting go of it which may decrease positioning errors what would otherwise occur due to involvement of human interaction. Coupling 114 may be adjusted to provide for a suitable stiffness to keep first arm 110 stationary in case an operator lets go of device 112 or first arm 110.

According to the invention, connecting unit 116 may be slidably movable along first arm 110 to enable adjustment of the first counterweight arrangement. Additionally or alternatively, connecting unit 116 may enable an operator to adjust the counterweighting by fixing the cabling 118 at different points moving more or less of the cabling to one or the other side of connecting unit 116. Adjusting the counterweight may be useful in case devices 112 of differing weights are used, for example if coils of different weights are used. Adjusting the counterweight may also be useful in case operators with different preferences use the apparatus. Connecting unit 116 may be disposed at an end of the first arm that is opposite to the end of the first arm where the device attachment portion and/or device is disposed.

Alternatively to cabling 118, first arm 110 may be furnished with a different counterweight, such as for example a weight attached to first arm 110 to counter the weight of device 112. In some embodiments, first arm may be furnished with a display screen, or a combination of display screen and at least part of cabling 118 acting as a counterweight to device 112. The display screen may be configured to display operational parameters of device 112 to an operator. In embodiments where cabling 118 is at least in part connected to such a display screen, the display screen may obtain information concerning the operating parameters directly from the cabling. Thus counterweighting may be provided by the display screen and attached cabling, and an operator is enabled to observe operational parameters of device 112 from the screen acting as counterweight.

Mounted on chair 160, or alternatively on headrest 170, is base 130. According to the invention, base 130 defines a curve, which may comprise that base 130 comprises a curved support or rail mounted or mountable on chair 160 or headrest 170. Chair 160 may comprise a back portion and a seat portion, and wherein a base may be attached to a portion of the chair, for example to the back portion. The length of the curve may proceed in a plane, in some embodiments the plane may be parallel to the plane defined by floor level, that is, horizontal. When the length of the curve proceeds in a single plane, the curve spans, that is defines, that plane. Second arm 120 is in the example of FIGURE 1 mounted on base 130 in a movable way, for example so that the attachment point of second arm 120 to base 130 may be moved along the length of the curve. Moving the attachment point along the length of the curve may enable positioning of device 112 to different sides of the head. Moving the attachment point of second arm 120 to base 130 along the length of the curve may comprise moving the point along a rail that forms the curve of base 130. A rail in the form of a curve may be referred to as a rail in the shape of an arc. The ends of the arc may be attached at or near opposite corners of a top back portion of the chair. The second arm may remain in the same point of the curve in case the operator lets go of it. The base may be adjustably attached to a portion of the chair such that the base can be adjusted to a substantially horizontal orientation independently of the orientation of the portion of the chair to which it is attached. In some embodiments, counterweighting may be at least in part comprised of an actuator configured to move, or cause to move, the second arm along the curve and an actuator configured to lock at least the coupling

The movement of the attachment point of second arm 120 to base 130 may be motorized, for example where base 130 comprises a horizontal curved rail, a suitable electric motor may be employed to move second arm 120 along the length of the curve of the rail. In some embodiments, also coupling 114 may be operable by a suitable motor. In some embodiments, the first arm further comprises a device attachment portion configured to hold device 112. The device attachment portion may be connected to the first arm by a first joint or first joints which allow movement of the device attachment portion in one, two or three degrees of freedom with respect to the first arm. This may allow, for example, rotating device 112. The first joint or joints may be driven by suitable motors, for example electric motors, to provide for automated and highly reliable positioning of device 112. The first joint or first joints may be selectively lockable and/or their mobility may be adjustable with respect to at least one degree of movement between the first arm and the device attachment portion.

Mounting base 130 on chair 160 facilitates obtaining improved results from TMS or radiation therapy, as inadvertent movement of chair 160 will not result in movement of device 112 relative to the head. This is in contrast to solutions where the chair and the device are supported on a floor independently of each other. In these solutions, if either the chair or the device is inadvertently moved, the device will move relative to the head. Mounting base 130 on chair 160 also provides the advantage that the first and second arms may be built shorter, which inherently increases the accuracy of pointing as longer parts are more difficult to position accurately. Base 130 may be mounted on a back portion of the chair, for example. Alternatively, if base 130 is mounted on a headrest portion of the chair, the device may remain essentially immobile with respect to a head resting on the headrest in case the headrest is adjusted. A camera may be mounted on a back portion or headrest portion of the chair. Such a camera may be used to position device 112 in a correct place with respect to the head, for example by employing machine-readable visual cues attached to the head and device 112. Mounted on the chair, the camera does not move relative to the head in case the chair moves. Mounted on the headrest, the camera does not move with respect to the head in case the headrest is adjusted. In general, instead of a camera, an imaging and/or tracking device may be used.

Second arm 120 may be mounted on base 130 in a rotatable way, so that in addition to being able to be moved along the length of the curve, the angle between second arm 120 and a plane spanned by the shape of the curve may be modified. This may be achieved by providing a joint or hinge in a mechanism attaching second arm 120 to base 130. Such rotation may provide for a tilt of the apparatus comprising the first arm, second arm and device 120 toward and away from the head. To stabilize this rotation, a second counterweight may be provided, which is illustrated in FIGURE 1 as counterweight 140.

Counterweight 140 is suspended below base 130 and connected to second arm 120 by means of rigid third arm 150. Third arm 150 may be fixed to second arm 120 near the attachment point of second arm 120 to base 130, for example. Counterweight 140 may provide the benefit that also regarding the tilt of second arm 120, when an operator releases his grip of device 112, device 112 remains in place and doesn't move. To ensure this happens, this tilt of second arm 120 may be arranged with a suitable stiffness. The position of counterweight 140 on third arm 150 may be adjustable to provide for uniform counterweighting at different tilt angles.

Although illustrated as a separate weight, in some embodiments counterweight 140 is arranged using cabling 118. In some embodiments, cabling 118 is used to both provide the counterweighting at connecting unit 116 and counterweight 140. When cabling 118 is used in counterweight 140, counterweight 140 may comprise a connecting unit through which cabling 118 may traverse, and the connecting unit may allow an operator to adjust the counterweighting by fixing the cabling 118 at different points moving more or less of the cabling to one or the other side of the connecting unit.

As a result of the first counterweight provided at connecting unit 116 and second counterweight 140, device 140 may be stabilized in the sense that it is light to move in at least two degrees of freedom and additionally will remain in place in case an operator releases his grip of device 112. Locking movable couplings, hinges or joints requires less force when the apparatus is counterweighted with respect to the coupling, hinge or joint in question. Operating device 112 may become possible with one hand when device 112 is counterweighted.

Using at least one counterweight and/or lockable couplings, bearings, hinges and joints facilitates obtaining improved results from TMS or radiation therapy, as inadvertent movement of device 112 may be reduced or avoided. In addition to improved results, sessions of TMS or radiation therapy may be shorter in duration where positioning is improved by providing a stabler device 112. In addition or alternatively to being lockable, any couplings, bearings, hinges and joints may be capable of providing adjustable or partly restricted movement. Adjustable or partly restricted movement may comprise, for example, that movement requires more force.

Movable parts of the apparatus of FIGURE 1 may be lockable from a single actuator. In this case, an operator may use one hand to place device 112 to a desired position and activate the locking actuator with his other hand to lock device 112 into place. Said movable parts, comprising for example coupling 114, attachment of second arm 120 to base 130 and tilt of second arm 120, may be locked with electrical servos, solenoids, brake wires or linear actuators, for example.

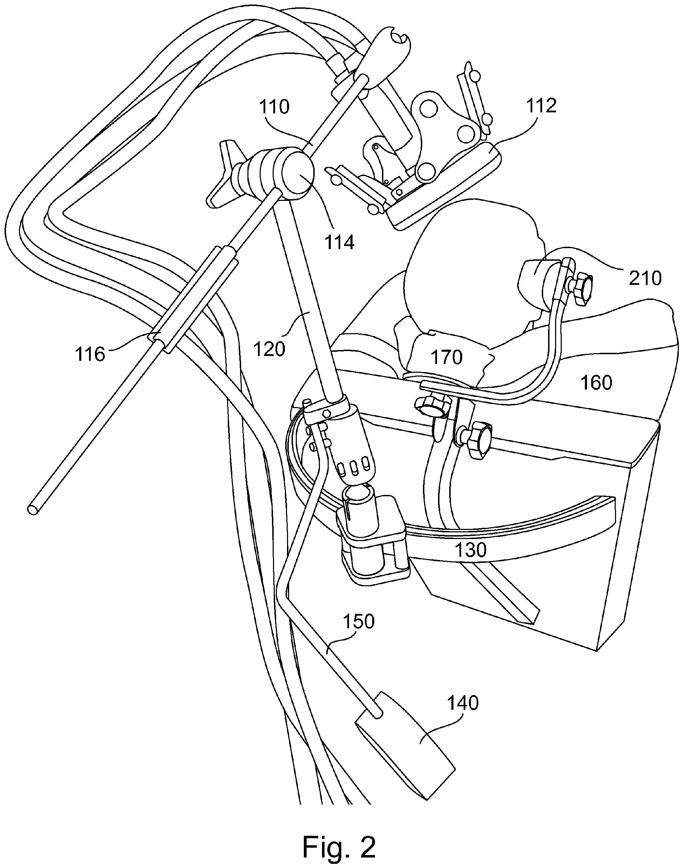

FIGURE 2 illustrates the example apparatus of FIGURE 1 from a different perspective. Like numbers denote like structures as in FIGURE 1. Head support 210, which was absent in FIGURE 1, is present in FIGURE 2 since it is visible from the perspective used in FIGURE 2. Head support 210 provides for stabilizing the head further on headrest 170. In some embodiments head supports 210 are provided, one on each side of the head to help immobilize the head. In these cases, at least one of the two head supports 210 may be adjustable. Head support 210 may comprise a sanitary, soft surface for hygienically, safely and comfortably supporting a head.

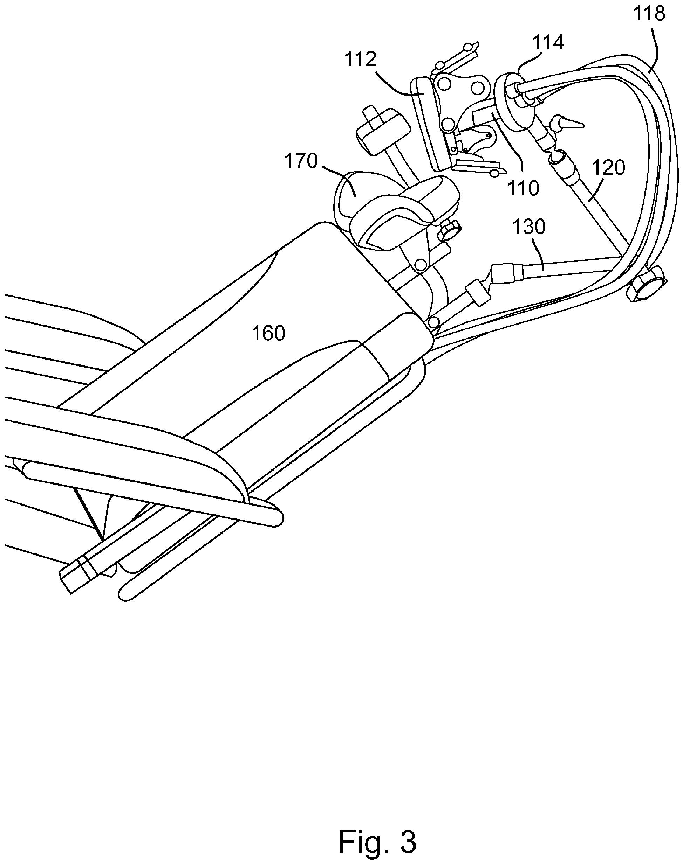

FIGURE 3 illustrates another example apparatus in accordance with at least some embodiments of the invention. Like numbers denote like structures as in FIGUREs 1 and 2. A difference to the embodiment illustrated in FIGURE 1 is in the structure of base 130. Instead of a curved structure, in the embodiment of FIGURE 3 base 130 takes the form of a base arm 130. Base arm 130 is rotatably attached to chair 160 so that the end of base arm 130 that is not attached to chair 160 can sweep a curve, thus defining the curve. The swept curve may be in a plane parallel to floor level, for example. Second arm 120 is in this embodiment attached to or near the end of base arm 130 that can sweep the curve, thus enabling second arm 120 to move in a similar way to the second arm 120 in the embodiment illustrated in FIGURE 1. Also in the embodiment of FIGURE 3, second arm 120 may be enabled to tilt with respect to a plane spanned by the curve. Also in the embodiment of FIGURE 3, counterweights may be provided as in FIGURE 1 to the tilt of second arm 120 with respect to a plane spanned by the curve and the movement of first arm 110 with respect to second arm 120 provided by coupling 114.

Although described above in terms of a chair, in some embodiments base 130 may be mounted or mountable on a bed, such as for example a hospital bed. Base 130 may be attached to a portion of a frame of the bed, for example. This provides the benefit that persons who are in too poor a condition to sit in a chair may be treated with the device.

In general there is provided an apparatus, comprising a first arm configured to accept a device, the first arm comprising a first counterweight arrangement, a second arm supporting the first arm by a coupling, the second arm optionally supporting a second counterweight arrangement, and a base defining a curve and having the second arm mounted thereon, wherein the second arm is movable along the curve, the base configured to be mounted on a chair.

The device may comprise a TMS coil, for example. The first counterweight arrangement may comprise a connecting unit enabled to accept attachment of cabling used to power and/or control the device, for example.

The second counterweight arrangement may comprise a connecting unit enabled to accept attachment of cabling used to power and/or control the device, for example. In first and second counterweight arrangements may both be enabled to accept attachment of cabling used to power and/or control the device to provide counterweighting. Alternatively, at least one of the first and second counterweight arrangements may comprise a weight other than the cabling used to power and/or control the device. At least one of the first and second counterweight arrangements may comprise both cabling and a separate weight.

The base may be at least in part formed in the shape of the curve. Alternatively, the base may comprise a base arm, the base arm defining the curve by being at one end rotatably fixed to the chair and the curve being formed by movement of the other end. Where the base is formed in the shape of a curve, it may comprise a rail. Such a rail may be mounted on a chair or bed at two points, for example at both ends. Such a rail may be semicircular or semi-elliptical in shape, for example.

The coupling may be configured to provide a rotating or sliding movement for the first arm with respect to the second arm. The coupling may comprise a ball joint.

The second counterweight arrangement may comprise a weight arranged below the base by a third arm, the third arm being mounted at one end on the second arm and the third arm supporting the weight at the other end. The second counterweight arrangement may counterweight a tilting of the second arm with respect to a plane spanned by the shape of the curve.

The first arm, second arm, third arm, base and/or base arm may be constructed of a suitable rigid material to allow for reliable positioning of device 112 when moving parts are locked. For example, the arms may be metallic pipes built of stainless steel or aluminum.

In some embodiments at least one of the first arm, second arm, third arm, base and/or base arm are constructed of non-magnetic material to minimize any effects on a shape of a magnetic field generated by device 112. Examples of suitable non-magnetic materials include plastic and graphite.

In one embodiment, a system for Transcranial magnetic stimulation, TMS, is provided, comprising a TMS coil device having at least one cable extending therefrom, a first arm having a device attachment portion at one end configured to lockably receive the TMS coil device, said first arm further having a cable attachment portion configured to receive the at least one cable extending from the TMS coil device, a second arm supporting the first arm by a coupling, and a base defining a curve and having the second arm mounted thereon, wherein the second arm is movable along the curve, said base configured to be mounted on a portion of a chair or bed.

Specific variants of this embodiment may comprise at least one feature from the following bulleted list:
- the device attachment portion is configured to hold the device in a locked position and orientation with respect to the device attachment portion
- the device attachment portion is connected to the first arm by a first joint, or first joints, which allow movement of the device attachment portion in three degrees of freedom with respect to the first arm
- the first joint or first joints are selectively lockable and/or their mobility is adjustable with respect to at least one degree of movement between the first arm and the device attachment portion
- the coupling is configured to provide more than one degree of freedom for the first arm with respect to the second arm
- the coupling is selectively lockable and/or its mobility is adjustable with respect to at least one degree of movement between the first arm and the second arm
- the cable attachment portion is capable of acting as a counterweight to the TMS coil device when the TMS coil device is lockably attached to the first arm and the at least one cable extending from the TMS coil device is attached to the cable attachment portion on the first arm
- the cable attachment portion is adjustable along at least a portion of a length of the first arm
- the cable attachment portion is located at an end of the first arm opposite from the device attachment portion
- the base comprises a rail in the shape of an arc, wherein the ends of the arc are attached at or near opposite corners of a top back portion of the chair
- the second arm is moveable along the length of the arc
- the system further comprises a counterweight portion attached to the second arm and located at least partially below the base
- the system further comprises a chair with a back portion and a seat portion, and wherein the base is attached to a portion of the chair
- the base is attached to a back portion of the chair in a horizontal orientation
- the chair further comprises an adjustable headrest and the base is attached to the headrest
- the base is adjustably attached to a portion of the chair such that the base portion can be adjusted to a substantially horizontal orientation independently of the orientation of the portion of the chair to which it is attached
- the system further comprises a bed with a frame, and wherein the base is attached to a portion of the frame.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made.

## Claims

1. An apparatus, comprising:
a first arm (110) configured to accept a transcranial magnetic stimulation device (112), the first arm (110) comprising a first counterweight arrangement (116);
a second arm (120) supporting the first arm by a coupling (114),
**characterized by**
a base (130) defining and being formed in a shape of a non-straight curve and having the second arm (120) mounted thereon at an attachment point, wherein the attachment point is movable along the non-straight curve to enable positioning the transcranial magnetic stimulation device (112) to different sides of a head, the base (130) configured to be mounted on a chair (160).

2. An apparatus according to claim 1, wherein the second arm (120) supports a second counterweight arrangement (140).

3. An apparatus according to any of claims 1 - 2, wherein the first counterweight arrangement (116) comprises cabling of the device attached to the first arm (110), the coupling (114) being between the transcranial magnetic stimulation device (112) and the section of the first arm (110) where the cabling is attached.

4. An apparatus according to any preceding claim, wherein the first counterweight arrangement (116) comprises at least one of a display screen, an actuator configured to move, or cause to move, the second arm (120) along the curve and an actuator configured to lock at least the coupling (114).

5. An apparatus according to claim 3 or 4, wherein the cabling is attached to the first arm (110) via a connecting unit, the connecting unit being slidably movable along the first arm to enable adjustment of the first counterweight arrangement (116).

6. An apparatus according to any preceding claim, wherein the coupling (114) is configured to provide a rotating or sliding movement for the first arm (110) with respect to the second arm (120).

7. An apparatus according to any of claims 2 - 6, wherein the second counterweight arrangement (140) comprises a weight arranged below the base by a third arm (150), the third arm (150) being mounted on the second arm (120) and the third arm (150) supporting the weight (140) at the other end.

8. An apparatus according to claim 7, wherein the coupling (114) and the mounting of the second arm on the base are lockable with at least one actuator.

9. An apparatus according to any of claims 2 - 8, wherein the second arm (120) is tiltable with respect to a plane spanned by the length of the curve and the second counterweight arrangement (140) counterweights the tilt.

10. An apparatus according to any preceding claim, wherein the base (130) is configured to be mounted on a back portion of a chair (160).

11. An apparatus according to any of claims 1 - 10, wherein the base (130) is configured to be mounted on a headrest of a chair (160).

12. An apparatus according to any of claims 1, 3, 4, 5, 6, 7, 8, 9, 10 or 11, wherein the base (160) comprises a rail.

13. A chair (160) comprising an apparatus according to at least one of claims 1 - 12.

## Patentansprüche

1. Einrichtung, umfassend:
einen ersten Arm (110), der konfiguriert ist, um eine transkranielle magnetische Stimulationsvorrichtung (112) aufzunehmen, wobei der erste Arm (110) eine erste Gegengewichtanordnung (116) umfasst;
einen zweiten Arm (120), der den ersten Arm durch eine Kopplung (114) trägt,
**gekennzeichnet durch**
eine Basis (130), die eine Form einer nicht geradlinigen Biegung definiert und als diese geformt ist, und die den zweiten Arm (120) darauf an einer Befestigungsstelle montiert aufweist, wobei die Befestigungsstelle entlang der nicht geradlinigen Biegung bewegbar ist, um das Positionieren der transkraniellen magnetischen Stimulationsvorrichtung (112) zu unterschiedlichen Seiten eines Kopfs zu ermöglichen, wobei die Basis (130) konfiguriert ist, um auf einen Sessel (160) montiert zu werden.

2. Einrichtung nach Anspruch 1, wobei der zweite Arm (120) eine zweite Gegengewichtanordnung (140) trägt.

3. Einrichtung nach einem der Ansprüche 1-2, wobei die erste Gegengewichtanordnung (116) die Verkabelung der Vorrichtung, die an dem ersten Arm (110) angebracht ist, umfasst, wobei das Kopplung (114) zwischen der transkraniellen magnetischen Stimulationsvorrichtung (112) und der Sektion des ersten Arms (110), an der die Verkabelung angebracht ist, liegt.

4. Einrichtung nach einem der vorstehenden Ansprüche, wobei die erste Gegengewichtanordnung (116) mindestens einen eines Anzeigebildschirms, eines Aktuators, der konfiguriert ist, um den zweiten Arm (120) entlang der Biegung zu bewegen oder zum Bewegen zu veranlassen, und einen Aktuator, der konfiguriert ist, um die mindestens eine Kopplung (114) zu verriegeln, umfasst.

5. Einrichtung nach Anspruch 3 oder 4, wobei die Verkabelung an dem ersten Arm (110) über eine Anschlusseinheit angebracht ist, wobei die Anschlusseinheit gleitbar entlang des ersten Arms bewegbar ist, um das Einstellen der ersten Gegengewichtanordnung (116) zu ermöglichen.

6. Einrichtung nach einem der vorstehenden Ansprüche, wobei die Kopplung (114) konfiguriert ist, um eine Dreh- oder Gleitbewegung für den ersten Arm (110) in Bezug zu dem zweiten Arm (120) bereitzustellen.

7. Einrichtung nach einem der Ansprüche 2-6, wobei die zweite Gegengewichtanordnung (140) ein Gewicht umfasst, das unter der Basis durch einen dritten Arm (150) eingerichtet ist, wobei der dritte Arm (150) auf dem zweiten Arm (120) montiert ist, und der dritte Arm (150) das Gewicht (140) an dem anderen Ende trägt.

8. Einrichtung nach Anspruch 7, wobei die Kopplung (114) und die Montage des zweiten Arms auf der Basis mit mindestens einem Aktuator verriegelbar sind.

9. Einrichtung nach einem der Ansprüche 2-8, wobei der zweite Arm (120) in Bezug zu einer Ebene kippbar ist, die von der Länge der Biegung überspannt wird, und die zweite Gegengewichtanordnung (140) das Gegengewicht zu dem Kippen ist.

10. Einrichtung nach einem der vorstehenden Ansprüche, wobei die Basis (130) konfiguriert ist, um auf einen Rückseitenabschnitt eines Sessels (160) montiert zu werden.

11. Einrichtung nach einem der Ansprüche 1-10, wobei die Basis (130) konfiguriert ist, um auf eine Kopfstütze eines Sessels (160) montiert zu werden.

12. Einrichtung nach einem der Ansprüche 1, 3, 4, 5, 6, 7, 8, 9, 10 oder 11, wobei die Basis (160) eine Schiene umfasst.

13. Sessel (160), der eine Einrichtung nach einem der Ansprüche 1-12 umfasst.

## Revendications

1. Appareil, comprenant :
un premier bras (110) configuré pour accepter un dispositif de stimulation magnétique transcrânienne (112), le premier bras (110) comprenant un premier agencement de contrepoids (116) ;
un deuxième bras (120) supportant le premier bras par un couplage (114),
**caractérisé par**
une base (130) définissant et étant formée selon une forme d'une courbe non droite et ayant le deuxième bras (120) monté sur celle-ci au niveau d'un point de fixation, dans lequel le point de fixation est mobile le long de la courbe non droite pour permettre le positionnement du dispositif de stimulation magnétique transcrânienne (112) sur différents côtés d'une tête, la base (130) étant configurée pour être montée sur une chaise (160).

2. Appareil selon la revendication 1, dans lequel le deuxième bras (120) supporte un second agencement de contrepoids (140).

3. Appareil selon l'une quelconque des revendications 1 et 2, dans lequel le premier agencement de contrepoids (116) comprend un câblage du dispositif fixé au premier bras (110), le couplage (114) étant entre le dispositif de stimulation magnétique transcrânienne (112) et la section du premier bras (110) où le câblage est fixé.

4. Appareil selon l'une quelconque des revendications précédentes, dans lequel le premier agencement de contrepoids (116) comprend au moins l'un parmi un écran d'affichage, un actionneur configuré pour déplacer, ou amener à se déplacer, le deuxième bras (120) le long de la courbe et un actionneur configuré pour verrouiller au moins le couplage (114).

5. Appareil selon la revendication 3 ou 4, dans lequel le câblage est fixé au premier bras (110) via une unité de connexion, l'unité de connexion étant mobile de manière coulissante le long du premier bras pour permettre un ajustement du premier agencement de contrepoids (116).

6. Appareil selon l'une quelconque des revendications précédentes, dans lequel le couplage (114) est configuré pour fournir un déplacement rotatif ou coulissant pour le premier bras (110) par rapport au deuxième bras (120).

7. Appareil selon l'une quelconque des revendications 2 à 6, dans lequel le second agencement de contrepoids (140) comprend un poids agencé sous la base par un troisième bras (150), le troisième bras (150) étant monté sur le deuxième bras (120) et le troisième bras (150) supportant le poids (140) au niveau de l'autre extrémité.

8. Appareil selon la revendication 7, dans lequel le couplage (114) et le montage du deuxième bras sur la base peuvent être verrouillés avec au moins un actionneur.

9. Appareil selon l'une quelconque des revendications 2 à 8, dans lequel le deuxième bras (120) peut être incliné par rapport à un plan étendu de la longueur de la courbe et le second agencement de contrepoids (140) contrebalance l'inclinaison.

10. Appareil selon l'une quelconque des revendications précédentes, dans lequel la base (130) est configurée pour être montée sur une partie arrière d'une chaise (160).

11. Appareil selon l'une quelconque des revendications 1 à 10, dans lequel la base (130) est configurée pour être montée sur un appui-tête d'une chaise (160).

12. Appareil selon l'une quelconque des revendications 1, 3, 4, 5, 6, 7, 8, 9, 10 ou 11, dans lequel la base (160) comprend un rail.

13. Chaise (160) comprenant un appareil selon au moins l'une des revendications 1 à 12.
